(19) Europäisches Patentamt
European Patent Office
Office européen des brevets

(11) EP 1 874 410 B1

(12) **FASCICULE DE BREVET EUROPEEN**

(45) Date de publication et mention de la délivrance du brevet:
**11.03.2009 Bulletin 2009/11**

(21) Numéro de dépôt: **06754908.9**

(22) Date de dépôt: **27.04.2006**

(51) Int Cl.:
***A61Q 19/02*** *(2006.01)* ***A61K 8/49*** *(2006.01)*
***A61K 31/42*** *(2006.01)*

(86) Numéro de dépôt international:
**PCT/EP2006/061902**

(87) Numéro de publication internationale:
**WO 2006/114443 (02.11.2006 Gazette 2006/44)**

(54) **COMPOSITION COSMETIQUE DEPIGMENTANTE OU ECLAIRCISSANTE COMPRENANT AU MOINS UNE OXAZOLINE A TITRE DE PRINCIPE ACTIF**

ENTPIGMENTISIERENDE BZW. AUFHELLENDE KOSMETIKZUSAMMENSETZUNG, DIE MINDESTENS EIN OXAZOLIN ALS WIRKSTOFF UMFASST

DEPIGMENTING OR BRIGHTENING COSMETIC COMPOSITION COMPRISING AT LEAST ONE OXAZOLIN AS AN ACTIVE INGREDIENT

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **27.04.2005 FR 0504227**

(43) Date de publication de la demande:
**09.01.2008 Bulletin 2008/02**

(73) Titulaire: **Laboratoires Expanscience
92400 Courbevoie (FR)**

(72) Inventeurs:
- **PICCARDI, Nathalie
F-21310 Arceau (FR)**
- **MSIKA, Philippe
F-78000 Versailles (FR)**

(74) Mandataire: **Ahner, Francis et al
Cabinet Régimbeau,
20, rue de Chazelles
75847 Paris Cedex 17 (FR)**

(56) Documents cités:
**FR-A- 2 834 216**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention se rapporte à l'utilisation d'une composition cosmétique à action dépigmentante ou éclaircissante du teint, comprenant, à titre de principe actif, au moins une oxazoline.

La couleur de la peau est due à plusieurs substances : l'hémoglobine des vaisseaux, les caroténoïdes du derme et, surtout, la mélanine de l'épiderme. Cette mélanine est produite par les mélanocytes de la couche basale, sous l'action de la tyrosinase, du cuivre et de l'oxygène.

La mélanine de la peau est formée par une association complexe d'eumélanine et de phéomélanine.

Leurs biosynthèses sont communes jusqu'à la dopaquinone (double oxydation de la tyrosine par la tyrosinase, enzyme cupro-protéique). Leur chemin ensuite diverge.

L'eumélanine brune est un polymère d'indole-5-6-quinone tandis que la phéomélanine responsable de la couleur rousse est un composé contenant près de 10% de soufre et de structure polymère de la cystéinyl dopa.

D'autres enzymes que la tyrosinase participent à la genèse et au contrôle des mélanines : la dopachrome oxydo-réductase, l' $\alpha$-glutamyl transpepsidase, le système glutathion (réductase-péroxydase), la dopachrome tautomérase.

Sous l'effet de stimulations exogènes ou endogènes, il peut apparaître des modifications de la teinte de la peau : ce sont les dyschromies (hyperchromie et hypochromie).

Ces modifications peuvent siéger dans l'épiderme ou dans le derme et être dues à une variation de la quantité de mélanine ou du nombre de mélanocytes.

Les hyperchromies sont des accumulations de pigments mélaniques, de caroténoïdes ou de pigments exogènes.

Parmi les hyperchromies, nous pouvons citer le mélasma, qui est défini comme une hypermélanose acquise du visage qui peut s'observer dans les deux sexes et dans toutes les races. Le mélasma apparaît plus fréquemment chez les femmes utilisant une contraception orale ou pendant la grossesse (masque de grossesse, chloasma).

Le masque de grossesse, ou chloasma, apparaît chez les femmes qui ont un taux d'hormones féminines important et dont la peau est exposée au soleil. Il touche ainsi principalement les femmes enceintes ou les femmes prenant une pilule contraceptive. Il prend la forme de plaques pigmentées de couleur marron, souvent symétriques, de forme plus ou moins régulière.

Le vieillissement cutané se caractérise également par l'apparition de tâches pigmentaires. On parle alors de lentigo solaires pour les zones les plus fréquemment photo-exposées (visage, mains, décolleté), et de lentigo séniles, tâches pigmentaire assez larges, qui apparaissent chez le sujet âgé au niveau des mains, du visage et des bras.

Les agents dépigmentants ou blanchissants du teint sont des composés chimiques capables d'agir à l'échelon tissulaire, cellulaire ou subcellulaire. Ils agissent sur la formation, le transport, la couleur de la mélanine elle-même ou sur la viabilité du mélanocyte (mélanocytotoxicité).

D'autre part, il est nécessaire de mettre en évidence et de supprimer le facteur induisant l'hyperpigmentation avant de la traiter (U.V., parfum, oestroprogestatif) et de conseiller une protection solaire type protection maximale tout au long du suivi médical.

Enfin, il est possible d'éliminer les couches superficielles de cornéocytes contenant de la mélanine, et ainsi réaliser une dépigmentation physique de surface, traitement qui favorise par ailleurs la pénétration des agents dépigmentants.

Les motivations qui poussent à décolorer la peau peuvent être diverses. L'éclaircissement franc du teint est recherché en Afrique Noire avec des solutions traditionnelles ou chimiques qui présentent des effets secondaires néfastes importants sur l'aspect et la structure de la peau. La pâleur ou la blancheur du visage asiatique est obtenue avec des molécules agissant avec le moins de toxicité possible (l'arbutine, l'acide kojique, l'acide ascorbique).

Le traitement des taches d'hyperpigmentation des sujets blancs fait appel à des molécules diverses dont la principale, l'hydroquinone, fait l'objet d'une surveillance accrue et dont la dose maximale en cosmétique est de 2%.

Il existe donc un besoin pour des compositions présentant une activité dépigmentante ou éclaircissante et qui soient bien tolérées par la peau.

C'est pourquoi la présente invention a pour objet l'utilisation d'une composition dermatologique et/ou cosmétique, caractérisée en ce qu'elle contient au moins une oxazoline, à titre de principe actif dépigmentant ou éclaircissant du teint.

Dans la demande de brevet français FR 2 834 216, les inventeurs décrivent l'utilisation d'oxazolines, qui permettent l'inhibition de la migration des cellules de Langerhans. Cette demande décrit ainsi un médicament, comprenant au moins une oxazoline en tant que principe actif, destiné au traitement ou à la prévention des réactions allergiques, et/ou inflammatoires, et/ou irritatives de la peau et/ou des muqueuses.

Dans la demande brevet français FR 2 856 294, les inventeurs décrivent l'utilisation d'oxazolines pour inhiber la lipogenèse dans des adipocytes humains. Cette demande décrit ainsi l'utilisation d'une composition comprenant au moins une oxazoline, à titre de principe actif, en tant que composition amincissante, et/ou pour prévenir et/ou traiter la

cellulite.

La demande de brevet US 4 876 249 décrit des compositions comprenant des oxazolines, dans lesquelles les oxazolines sont des promoteurs de pénétration d'agents actifs physiologiques à travers la couche *stratum corneum* de la peau.

D'une manière surprenante, les inventeurs ont maintenant mis en évidence que les oxazolines présentent également une action dépigmentante. Les inventeurs ont ainsi mis au point une composition cosmétique et/ou dermatologique dépigmentante et/ou à action éclaircissante comprenant au moins une oxazoline, à titre de principe actif dépigmentant.

L'invention a donc pour objet l'utilisation d'au moins une oxazoline, à titre de principe actif dépigmentant, dans une composition dépigmentante. La composition dépigmentante est avantageusement destinée à réduire et/ou supprimer et/ou prévenir les taches de pigmentation ou à éclaircir la peau naturellement pigmentée.

Les oxazolines selon la présente invention répondent avantageusement aux formules générales suivantes:

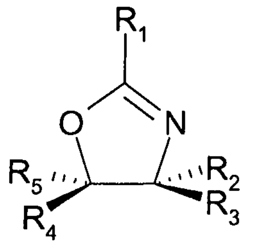

Type 1

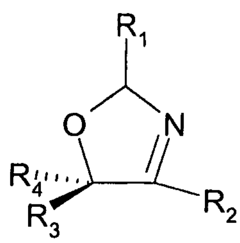

Type 2

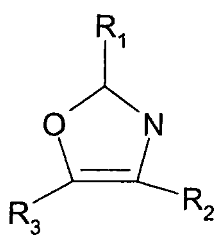

Type 3

dans laquelle $R_1$ représente un groupe alkyle en $C_1$-$C_{40}$, linéaire ou ramifié, saturé ou insaturé, comprenant éventuellement une ou plusieurs insaturation(s) éthylénique(s) ainsi que un ou plusieurs substituant(s) choisi(s) dans le groupe formé par les radicaux hydroxy (OH) et alcoxy en $C_1$-$C_6$ ($OC_1$-$C_6$) ; $R_2$, $R_3$, $R_4$ et $R_5$ représentent, de manière indépendante, un atome d'hydrogène, un radical hydroxy, ou un groupe alkyle en -$C_1$-$C_{30}$, linéaire ou ramifié, saturé ou insaturé, comprenant éventuellement une ou plusieurs insaturations éthyléniques ainsi que un ou plusieurs substituant(s) choisi (s) dans le groupe formé par les radicaux hydroxy (OH), alcoxy en $C_1$-$C_6$ ($OC_1$-$C_6$) et alcoxy en $C_1$-$C_6$ carbonyles ($COOC_1$-$C_6$). Par le terme de "alcoxy en $C_1$-$C_6$ ($OC_1$-$C_6$)", on entend au sens de la présente invention, un radical alcoxy dont le groupement alkyle comprend de 1 à 6 atomes de carbone.

$R_1$ représente avantageusement un groupe alkyle en $C_8$-$C_{30}$, encore plus avantageusement en $C_8$-$C_{20}$, linéaire ou ramifié, saturé ou insaturé, comprenant éventuellement une ou plusieurs insaturation(s) éthylénique(s) ainsi que un ou plusieurs substituant(s) choisi(s) dans le groupe formé par les radicaux hydroxy (OH) et alcoxy en $C_1$-$C_6$ ($OC_1$-$C_6$).

$R_2$, $R_3$, $R_4$ et $R_5$ représentent avantageusement, de manière indépendante, un atome d'hydrogène, un radical hydroxy, ou un groupe alkyle en $C_1$-$C_{10}$, encore plus avantageusement en $C_1$-$C_6$, linéaire ou ramifié, saturé ou insaturé, comprenant éventuellement une ou plusieurs insaturations éthyléniques ainsi que un ou plusieurs substituant(s) choisi(s) dans le groupe formé par les radicaux hydroxy (OH), alcoxy en $C_1$-$C_6$ ($OC_1$-$C_6$) et alcoxy en $C_1$-$C_6$ carbonyles ($COOC_1$-$C_6$).

Selon un mode avantageux de la présente invention, la dite oxazoline est une oxazoline de type 1 sélectionnée dans le groupe composé de la 2-undécyl-4-hydroxyméthyl-4-méthyl-1,3-oxazoline, de la 2-undécyl-4,4-diméthyl-1,3-oxazoline, de la (E)-4,4-diméthyl-2-heptadéc-8-ényl-1,3-oxazoline, de la 4-hydroxyméthyl-4-méthyl-2-heptadécyl-1,3-oxazoline, la (E)-4-hydroxyméthyl-4-méthyl-2-heptadéc-8-ényl-1,3-oxazoline, la 2-undécyl-4-éthyl-4-hydroxyméthyl-1,3-oxazoline. Avantageusement, la dite oxazoline est la 2-undécyl-4,4-diméthyl-1,3-oxazoline, appelée OX100, de formule:

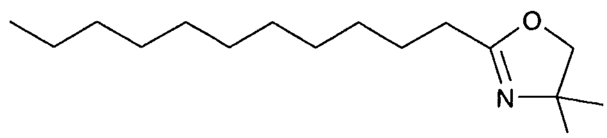

De nombreuses voies de synthèse sont connues pour préparer les oxazolines selon l'invention. Ainsi, celles-ci peuvent être préparées par synthèse chimique en faisant réagir un acide gras (ou un ester méthylique) et un amino-alcool, le plus souvent en présence d'un agent azéotropique afin de favoriser l'élimination de l'eau formée (et du méthanol formé). Une autre voie de synthèse possible consiste à condenser un halo-amide en présence d'une base forte ou de carbonate de sodium (R. M. Lusskin, J. Amer. Chem. Soc., 72, (1950), 5577). Les oxazolines peuvent également être synthétisées par réaction des époxydes sur les nitriles, par réaction du chlorure de thionyle sur les hydroxyamides ou encore, par action d'un acide sur une aziridinylphosphine.

La concentration en oxazoline dans la composition cosmétique et/ou dermatologique selon l'invention est avantageusement comprise entre environ 0,01 et environ 10% en poids, plus avantageusement entre environ 0,01 et environ 5% en poids, encore plus avantageusement entre environ 0,05 et environ 3% en poids, par rapport au poids total de la composition

La composition utilisée selon l'invention peut contenir d'autres actifs à action dépigmentante, conduisant à un effet complémentaire ou synergique. Les oxazolines peuvent être associées à des agents dépigmentants connus de l'homme de métier tels que l'hydroquinone et ses dérivés, l'arbutine, l'acide rétinoïque, le rétinol, le rétinaldéhyde, l'acide kojique, l'acide azélaïque, la vitamine B3 ou PP, les dérivés du résorcinol, le résvératrol, des extraits de licorice ou de murier blanc, l'acide alpha-lipoïque, l'acide linoléique, des chélateurs de cations tels que l'EDTA (acide éthylène diamine tétra acétique), les extraits de soja.

Les oxazolines peuvent également être associées à des agents anti-oxydants, conduisant à un effet complémentaire ou synergique. Comme exemple d'agents anti-oxydants, on peut notamment citer la vitamine C, la vitamine E, les polyphénols (notamment ceux extraits du thé vert ou de raisin ou de pin), les dérivés soufrés.

Les oxazolines peuvent également être associées à des agents dépigmentants tels que le Sepiwhite® (N-undecylenoyl-L-phénylalanine) commercialisé par la société Seppic, conduisant à un effet complémentaire ou synergique.

Selon un autre aspect de l'invention, les compositions cosmétiques et/ou dermatologiques selon l'invention contiennent également, éventuellement avec un effet de synergie, au moins un filtre ou un écran solaire UVB et UVA ; tels les écrans ou filtres minéraux et/ou organiques connus de l'homme du métier qui adaptera leur choix et leurs concentrations en fonction du degré de protection recherché.

Enfin, les compositions cosmétiques et/ou dennatologiques selon l'invention peuvent également contenir des agents exfoliants tels que les alpha-hydroxyacides et salicylique et les dérivés sous forme d'ester par exemple.

Enfin, les compositions cosmétiques et/ou dermatologiques selon l'invention peuvent également contenir des agents anti-inflammatoires ou apaisants, des agents désensibilisants cutanés tels que des AINS (Anti-inflammatoires non stéroïdiens), des dermocorticoïdes, des agonistes PPAR (peroxysme proliferator activated receptor : récepteur activé par les proliférateurs de peroxysomes), des dérivés de réglisse, de bisabolol, d'isoflavones (de soja par exemple) glycosilés ou non, du palmitoyléthanolamide, des insaponifiables à base de phytostérols et de vitamines E, des anti-COX et/ou LOX (inhibiteur de cyclo-oxygènase et/ou de lipoxydase), des eaux thermales, marines ou reconstituées à partir d'oligoéléments exogènes.

Selon une variante avantageuse de l'invention, la composition comprend en tant que principe actif dépigmentant une oxazoline avec un autre actif dépigmentant tel que le Sepiwhite®. La composition comprend avantageusement en outre un cocktail de vitamines (vitamine C et E) qui confère une action anti-radicalaire et permet d'inhiber le monoxyde d'azote (NO).

En effet, NO est impliqué dans la régulation de la pigmentation.

La composition dermatologique et/ou cosmétique selon l'invention comprend un support dermatologiquement et/ou cosmétiquement acceptable, c'est à dire un support compatible avec la peau. Elle peut avantageusement se

présenter sous toutes les formes galéniques normalement utilisées pour une application topique, notamment sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide, d'une dispersion d'huile dans une phase aqueuse à l'aide de sphérules (nanosphères, nanocapsules, vésicules lipidiques), d'un dispositif trans-dermique ou sous toute autre forme pour application topique.

Cette composition peut être plus ou moins fluide et avoir l'aspect d'une crème blanche ou colorée, d'une pommade, d'un lait, d'une lotion, d'un sérum, d'une pâte, d'une mousse ou d'un gel. Elle peut éventuellement être appliquée sur la peau sous forme d'aérosol. Elle peut également se présenter sous forme solide, et par exemple sous forme de stick. Elle peut aussi être appliquée au moyen d'un patch.

La composition selon l'invention peut contenir également les adjuvants habituels dans le domaine cosmétique, tels que les stabilisants, les conservateurs, les antioxydants, les solvants, les parfums, les agents chélateurs, les absorbeurs d'odeur, des filtres chimiques ou minéraux, des pigments minéraux, les tensioactifs, les polymères, les huiles de silicone et les matières colorantes.

L'invention a également pour objet une méthode de traitement cosmétique pour réduire et/ou supprimer les taches de pigmentation, caractérisée en ce que l'on applique par voie topique une composition comprenant au moins une oxazoline. Cette méthode de traitement cométique permet d'uniformiser le teint. La composition est avantageusement telle que définie précédemment.

Les tâches de pigmentation peuvent être sans limitation des tâches de vieillesse, des tâches U.V. induites ou des tâches de phototoxicité (parfum, médicament, toxique exogène, brûlure) ou des chloasmas.

L'invention a aussi pour objet une méthode de traitement cosmétique pour éclaircir la peau, caractérisée en ce que l'on applique par voie topique une composition comprenant au moins une oxazoline. La composition est avantageusement telle que définie précédemment.

Les propriétés dépigmentantes des oxazolines peuvent, selon un autre aspect de l'invention, conduire à l'utilisation d'au moins une oxazoline en tant que principe actif pour la préparation d'un médicament actif comme dépigmentant.

L'oxazoline utilisée pour la fabrication du médicament est avantageusement telle que définie précédemment. Elle peut être utilisée en association, éventuellement avec un effet de synergie, avec au moins un autre agent dépigmentant tel que défini précédemment et/ou au moins un filtre solaire organique ou minéral et/ou un agent anti inflammatoire.

Les modes d'administration, les posologies et les formes galéniques optimales des composés et compositions selon l'invention peuvent être déterminés selon les critères généralement pris en compte dans l'établissement d'un traitement cosmétique et/ou dermatologique, adapté à un patient comme par exemple le type de peau.

Les exemples suivants illustrent la présente invention.

**Exemple 1** : crème de soin dépigmentant n°1

| INGREDIENTS | % p/p |
|---|---|
| Eau | QSP 100 |
| Di-$C_{12}$-$_{13}$ Alkyl Malate | 10,000 |
| Polydécène hydrogéné | 5,000 |
| Amidon d'Oryza Sativa | 4,000 |
| Alcool cétéaryl | 3,200 |
| Glycérine | 3,000 |
| Coco-glycérides hydrogénés | 3,000 |
| Stéarate de sorbitane | 3,000 |
| Undécylénoyl Phénylalanine | 2,000 |
| Glucoside ascorbyle | 2,000 |
| Trométhamine | 1,340 |
| Cérésine | 1,000 |

(suite)

| INGREDIENTS | % p/p |
|---|---|
| Glucoside cétéaryle | 0,800 |
| Parfum | 0,500 |
| Gomme xanthane | 0,400 |
| Cétyl phosphate de potassium | 0,400 |
| Gomme sclerotium | 0,300 |
| Hydroxyméthylglycinate de sodium | 0,200 |
| Oxazoline ou OX 100 | 0,100 |
| Tocophérol | 0,100 |

**Exemple 2** : crème de soin dépigmentant n˚2

| INGREDIENTS | % p/p |
|---|---|
| Eau | QSp 100 |
| Di-C12-13 Alkyl Malate | 10,000 |
| Polydécène hydrogéné | 5,000 |
| Amidon d'Oryza Sativa | 4,000 |
| Alcool cétéaryl | 3,200 |
| Glycérine | 3,000 |
| Coco-glycérides hydrogénés | 3,000 |
| Stéarate de sorbitane | 3,000 |
| Trométhamine | 1,340 |
| Cérésine | 1,000 |
| Glucoside cétéaryle | 0,800 |
| Parfum | 0,500 |
| Gomme xanthane | 0,400 |
| Cétyl phosphate de potassium | 0,400 |
| Gomme sclerotium | 0,300 |
| Hydroxyméthylglycinate de sodium | 0,200 |
| Oxazoline ou OX 100 | 0,200 |

**Exemple 3 :** Spray dépigmentant SPF (Suncreen Protection Factor : Facteur de protection écran solaire) 30

| Ingrédients | % p/p |
|---|---|
| Eau | QSP 100 |
| Tétraoctanoate de Pentaérythrityle | 15 à 30 |
| Dioxyde de titane | 1 à 10 |

(suite)

| Ingrédients | % p/p |
|---|---|
| Cyclométhicone | 1 à 10 |
| Oxyde de zinc | 1 à 10 |
| Méthylène Bis-Benzotriazolyl tétraméthylbutylphénol | 1 à 5 |
| Benzoate de ($C_{12}$-$C_{15}$)alkyle | 1 à 10 |
| 4,5,7-Trihydroxyisoflavone | 0,01 à 10 |
| Glycérine | 1 à 10 |
| Ether de dicaprylyle | 1 à 10 |
| Cyclopentasiloxane | 1 à 10 |
| Ethylhexyl diméthicone éthoxy glucoside | 1 à 10 |
| Propylène Glycol Dioctanoate | 1 à 10 |
| Chlorure de sodium | 1 à 5 |
| Copolymère PEG-45/Dodécyl Glycol | 1 à 5 |
| PEG-30 Dipolyhydroxystéarate | 1 à 5 |
| Huile d'insaponifiable de soja | 1 à 5 |
| Palmitate de dextrine | 1 à 5 |
| Oxazoline ou OX 100 | 0,01 à 3 |
| Extrait d'*aloé barbadensis* | 0,2 |
| Conservateurs | QS |
| Gluconate de zinc | 0,08 |

**Exemple 4 :** Crème dépigmentante SPF 50

| Ingrédients | % en poids |
|---|---|
| Eau | QSP 100 |
| Tétraoctanoate de Pentaérythrityle | 15 à 30 |
| Dioxyde de titane | 1 à 10 |
| Cyclométhicone | 1 à 10 |
| Oxyde de zinc | 1 à 10 |
| Benzoate de ($C_{12}$-$C_{15}$) alkyle | 1 à 10 |
| Undécylénoyl Phénylalanine | 0,5 à 2 |
| Oxazoline ou OX100 | 0,01 à 10 |
| 4,5,7-Trihydroxyisoflavone | 0,01 à 10 |
| Glycérine | 1 à 10 |
| Ether de dicaprylyle | 1 à 10 |
| Cyclopentasiloxane | 1 à 10 |
| Ethylhexyl diméthicone Ethoxy Glucoside | 1 à 10 |
| Propylène Glycol Dioctanoate | 1 à 10 |

(suite)

| Ingrédients | % en poids |
|---|---|
| Chlorure de sodium | 1 à 5 |
| Copolymère PEG-45/Dodécyl Glycol | 1 à 5 |
| PEG-30 Dipolyhydroxystéarate | 1 à 5 |
| Huile d'insaponifiable de soja | 1 à 5 |
| Palmitate de dextrine | 1 à 5 |
| Conservateurs | QS |
| Extrait d'*aloé barbadensis* | 0,2 |
| Gluconate de zinc | 0,08 |

**Exemple 5 :** Crème dépigmentante n°3

| INGREDIENTS | % p/p |
|---|---|
| Eau | QSP 100 |
| Di-$C_{12-13}$ Alkyl Malate | 10,000 |
| Polydécène hydrogéné | 5,000 |
| Amidon d'Oryza Sativa | 4,000 |
| Alcool cétéaryl | 3,200 |
| Glycérine | 3,000 |
| Coco-glycérides hydrogénés | 3,000 |
| Stéarate de sorbitane | 3,000 |
| Glucoside ascorbyle | 2,000 |
| Trométhamine | 1,340 |
| Cérésine | 1,000 |
| Glucoside cétéaryle | 0,800 |
| Parfum | 0,500 |
| Gomme xanthane | 0,400 |
| Cétyl phosphate de potassium | 0,400 |
| Bisabolol | 0,300 |
| Gomme sclérotium | 0,300 |
| Hydroxyméthylglycinate de sodium | 0,200 |
| Oxazoline ou OX 100 | 0,100 |
| Tocophérol | 0,100 |

**Exemple 6:** Evaluation de l'effet dépigmentant d'OX100

Volontaires

L'analyse des résultats a porté sur un panel de 21 volontaires adultes de sexe féminin, dont les caractéristiques sont présentées dans le tableau 1 suivant.

Tableau 1 : caractéristiques du panel de volontaires

| Age | Nature de la peau du visage | Peau "sensible" | "Atopiques" |
|---|---|---|---|
| 51 à 68 ans *(moy. : 60 ans)* | sèche : 57%<br>mixte sèche : 33%<br>mixte grasse : 10% | 24% | 19% |

Parmi ces volontaires, 14% étaient d'origine nord-africaine et 86% de type méditerranéen, dont 76% de phototype IV et 10% de phototype V. Tous ces volontaires présentaient des taches de pigmentation de type lentigo, au niveau des mains, et la majorité également au niveau du visage.

Résultats et discussion

**[0056]** * MESURES COLORIMETRIQUES *(Chromamètre™) :* On a vérifié l'homogénéité des variances et la normalité des distributions dans tous les cas.

⇒ *Au niveau de la zone sans tache*

Les mesures sont réalisées au niveau des mains, dans une zone sans tache de pigmentation. Les résultats correspondent aux moyennes et écarts par rapport à la moyenne - S.E.M. Ils sont donnés dans les tableaux 2 et 3 suivants.

Variable de Clarté - L*

Tableau 2 : variable de clarté, avant et après traitement, main traitée vs main témoin (zone sans tâche)

| MAINS | TEMOIN | TRAITEE | effet produit (Test "t" de Student) |
|---|---|---|---|
| T0 | 58,5 ± 0,6 | 58,4 ± 0,7 | 0,810 |
| T 6 semaines | 57,9 ± 0,6 | 59,2 ± 0,7 | 0,006 |
| effet temps (Test "t" de Student) | 0,173 | 0,080 | 0,017 |

Angle Typologique Individuel - I.T.A

Tableau 3 : angle typologique individuel,avant et après traitement, main traitée vs main témoin (zone sans tâche)

| MAINS | TEMOIN | TRAITEE | effet produit (Test "t" de Student) |
|---|---|---|---|
| T0 | 39,0 ± 2,3 | 38,5 ± 2,4 | 0,818 |
| T 6 semaines | 39,5 ± 2,8 | 44,1 ± 2,4 | 0,006 |
| effet temps (Test "t" de Student) | 0,760 | 0,000 | 0,008 |

Les résultats des effets temps et produit qui ont une valeur inférieure à 0,05 sont statistiquement significatifs. Le résultat correspondant à la fois à l'effet temps et à l'effet produit donne la valeur de la probabilité sur les différences Δ (T6 semaines- T0) par le test "t" de Student en série appariées (test de signification bilatérale, signif : $p < 0,05$).

On observe une augmentation statistiquement significative de la coordonnée de chromaticité L*, +2% ($p = 0,017$) ainsi que de l'angle I.T.A., +13% ($p = 0,008$) au niveau de la main traitée, après 6 semaines d'application, par rapport aux valeurs initiales et à celles obtenues sur la main témoin.

⇒ *Au niveau de la zone avec tache (mesure au niveau d'une tache de pigmentation préalablement repérée)*

Les mesures sont réalisées au niveau des mains, dans une zone avec tache de pigmentation. Les résultats correspondent aux moyennes et écarts par rapport à la moyenne - S.E.M. Ils sont donnés dans les tableaux 4 et 5 suivants.

Variable de Clarté - L*

Tableau 4 : variable de clarté, avant et après traitement, main traitée vs main témoin (zone avec tâche)

| MAINS | TEMOIN | TRAITEE | effet produit (Test "t" de Student) |
|---|---|---|---|
| T0 | 52,3 ± 1,0 | 51,2 ± 1,1 | 0,218 |
| T 6 semaines | 52,3 ± 0,9 | 52,4 ± 1,2 | 0,925 |
| effet temps (Test "t" de Student) | 0,986 | 0,022 | 0,048 |

Les résultats des effets temps et produit qui ont une valeur inférieure à 0,05 sont statistiquement significatifs. Le résultat correspondant à la fois à l'effet temps et à l'effet produit donne la valeur de la probabilité sur les différences Δ (T6 semaines- T0) par le test "t" de Student en série appariées (test de signification bilatérale, signif : $p < 0,05$).

Angle Typologique Individuel - I.T.A.

Tableau 5 : angle typologique individuel,avant et après traitement, main traitée vs main témoin (zone avec tâche)

| MAINS | TEMOIN | TRAITEE | effet produit (Test "t" de Student) |
|---|---|---|---|
| T0 | 10,4 ± 4,5 | 5,0 ± 4,5 | 0,150 |
| T 6 semaines | 11,6 ± 4,1 | 11,0 ± 5,2 | 0,869 |
| effet temps (Test "t" de Student) | 0,606 | 0,020 | 0,078 |

Les résultats des effets temps et produit qui ont une valeur inférieure à 0,05 sont statistiquement significatifs. Le résultat correspondant à la fois à l'effet temps et à l'effet produit donne la valeur de la probabilité sur les différences Δ (T6 semaines- T0) par le test "t" de Student en série appariées (test de signification bilatérale, signif : $p < 0,05$).

On observe une augmentation statistiquement significative de la coordonnée de chromaticité L*, +2% (p = 0,048) au niveau de la main traitée, après 6 semaines d'application, par rapport aux valeurs initiales et à celles obtenues sur la main témoin.

* EVALUATION CLINIQUE

⇒ *Au niveau des mains*

Les résultats de la variation statistiquement significative, après les 6 semaines d'application, de certains des critères évalués sont donnés dans le tableau 6 suivant :

Tableau 6 : variation, après traitement, au niveau des mains

| | TRAITEE |
|---|---|
| UNIFORMITE DE LA PEAU | + 10% *(p = 0,0209)* |
| ECLAT DE LA PEAU | + 16% *(p = 0,0002)* |
| INTENSITE DE LA TACHE DE PIGMENTATION | -5% *(p = 0,0143)* |

Les pourcentages correspondent aux variations par rapport aux évaluations initiales, la valeur de p correspond à la : probabilité p par rapport aux évaluations initiales (test de Wilcoxon, test de signification bilatérale, significativité : p < 0,05). Pour les mesures d'intensité de la tache de pigmentation, l'intensité de la tâche est préalablement repérée.

⇒ *Au niveau du visage*

Les résultats de la variation statistiquement significative, après les 6 semaines d'application, de certains des critères évalués sont donnés dans le tableau 7 suivant :

Tableau 7 : variation, après traitement, au niveau du visage

| | |
|---|---|
| UNIFORMITE DU TEINT | +4% *(p = 0,0455)* |
| ECLAT DU TEINT | +22% *(p = 0,0001)* |
| INTENSITE DE LA TACHE DE PIGMENTATION | -8% *(p = 0,0196)* |

Les pourcentages correspondent aux variations par rapport aux évaluations initiales, la valeur de p correspond à la : probabilité p par rapport aux évaluations initiales (test de Wilcoxon, test de signification bilatérale, significativité : p < 0,05). Pour les mesures d'intensité de la tache de pigmentation, l'intensité de la tâche est préalablement repérée.

* APPRECIATION GLOBALE DE L'ACCEPTABILITE ET DE L'EFFICACITE DU PRODUIT PAR LES VOLONTAIRES

57% des volontaires ont considéré que le produit a une acceptabilité qui peut être qualifiée de bonne à très bonne et 29% des volontaires ont considéré que le produit a une acceptabilité qui peut être qualifiée d'assez bonne.

38% des volontaires ont considéré que le produit a une efficacité en tant que soin dépigmentant / éclaircissant qui peut être qualifiée d'assez bonne à bonne.

* APPRECIATION DE L'ACCEPTABILITE CUTANEE PAR LES VOLONTAIRES

95% des volontaires ont déclaré ne pas avoir ressenti de manifestations cutanées et 100% des volontaires ont déclaré ne pas avoir ressenti de manifestations oculaires.

En conséquence, OX100 est un principe actif dépigmentant efficace, bien accepté dennatologiquement.

**Exemple 7 :** évaluation de l'effet dépigmentant d'une composition selon l'invention

On a étudié, l'efficacité cosmétologique dépigmentante, et la compatibilité cutanée clinique de la formule cosmétique selon l'invention dans le cadre de mélasma du visage.

Produits :

Crème dépigmentante : composition de l'exemple 1

Le produit est utilisé matin et soir au niveau des taches brunes, après la toilette, sur la peau sèche. La peau des personne testées est protégée des rayonnement solaire par application d'un écran total SPF60.

Pendant la durée de l'étude aucun autre produit dépigmentant n'est appliqué localement sur les zones concernées (cosmétiques ou médicament d'usage local).

I- Méthodes d'investigation

- auto-estimation par échelles analogiques visuelles, d'une durée de 60 jours, sous contrôle dermatologique, et
- étude biométrologique en ouvert avec mesures de l'index mélanique par Mexameter, Photographies standard et photographies UV

Ces évaluations et mesures ont été effectuées avant utilisation du produit, puis au bout de 60 jours.

Cette étude montre l'efficacité cosmétologique dépigmentante de la formule cosmétique de l'exemple 1 à visée dépigmentante, en comparant l'évolution des différentes composantes du mélasma.

Cette formule a été utilisée dans le cadre d'hyperchromies acquises à type de mélasma afin de contribuer à améliorer l'état cutané, en particulier en ce qui concerne l'effet sur la réduction de l'intensité de la couleur des zones hyperpigmentées.
La bonne acceptabilité cosmétologique et la tolérance allergologique de la formule ont été étudiées parallèlement.

* Principales caractéristiques du panel

Les volontaires entrant dans l'étude, au nombre de 20, toutes de sexe féminin, étaient âgées de 20 à 61 ans (âge moyen du panel 42,7 ans), ont été répartis en groupes d'âge par dizaine :

| | | |
|---|---|---|
| Carnation : | moyenne | 5 sujets |
| | mate | 14 sujets |
| | africaine | 1 sujet |
| Type de peau : | normale à tendance sèche | 5 sujets |
| | déshydratée sénescente | 2 sujets |
| | tendance sèche | 1 sujet |
| | grasse, mixte marquée | 3 sujets |
| | mixte marquée | 8 sujets |
| | grasse | 1 sujet |
| Réactivité cutanée : | augmentée | 3 sujets |
| | augmentée + érythrose | 1 sujet |
| | normale | 16 sujets |
| Phototype : | IV | 5 sujets |
| | V | 14 sujets |
| | Africain | 1 sujet |

Dans cette étude, n'ont été retenues que les lésions hyperpigmentées du visage de type de mélasma et ayant une durée d'évolution supérieure à 6 mois et inférieure à 10 ans. L'origine hormonale des lésions de mélasma est retrouvée de façon nette chez 9 des sujets ; elle est probable chez 10 sujets ; pour 1 sujet c'est une origine inflammatoire qui est probable.

* Etude clinique

Au début du traitement (J0), puis à J60 (après 60 jours de traitement), l'état clinique a été évalué en utilisant le scorage MASI (Melasma Area and Severity Index = surface du mélasme et indice de sévérité), tel que décrit par Kimbrough-Green et al. Arch Dermatol 1994; 130:727-733
Le calcul utilisé a été celui du MASI simplifié, c'est à dire :

$$(m\ MASI) = (D+H) \times A \qquad (Eq\ 1)$$

* Mesures biométrologiques

➢ L'index mélanique de la peau a été mesuré à l'aide d'un MEXAMETER, le diamètre de la surface de mesure étant de 5 mm.

L'index mélanique a été calculé suivant l'équation suivante :

$$IM = (500/\log 5) * (\log(\text{réflexion infrarouge}/\text{ réflexion rouge}) + \log 5) \qquad (Eq2)$$

L'index mélanique a été mesuré sur les zones de test à droite et à gauche et ceci avant toute utilisation de produit et au bout de 60 jours.

Zones de mesure : 2 zones hyperpigmentées et deux zones saines de chaque côté, les zones hyperpigmentées recevant le produit, et une zone de peau saine en périphérie de chaque zone hyperpigmentée.

> Photographies standard et UV

Des photographies standard, profil droit et profil gauche, ont été effectuées à chaque temps d'étude.
Des photographies U.V., profil droit et profil gauche, ont été effectuées à chaque temps d'étude et ont fait l'objet d'un traitement informatisé de la répartition des couleurs. Elles ont servi au repérage précis des zones hyperpigmentées.

➢ Tolérance

Une évaluation de la tolérance globale a été effectuée à J60 selon une échelle de sévérité à 4 niveaux:

➢ Evaluation globale de l'efficacité

A J60, un avis a été donné sur l'efficacité dépigmentante du produit utilisé en utilisant le barème suivant : Pas du tout satisfaisante, moyennement satisfaisante, satisfaisante, très satisfaisante.

➢ Auto estimation

A J60, un questionnaire a été posé au sujet concernant les qualités cosmétiques du produit testé. L'appréciation subjective psycho-sensorielle d'acceptabilité cosmétique concernant le produit et ses conditions d'utilisation a été effectuée grâce à des questions posées dans un questionnaire administré à chaque sujet volontaire à J60.

II) Analyse des résultats ($60^{ème}$ jour = T1) :

A) Etude clinique par scorage MASI

Le suivi de l'état cutané a été effectué, à partir de l'état initial constaté lors de l'entrée dans l'étude, par le dermatologue après 60 jours d'utilisation du produit testé.
Initialement la moyenne des scores MASI pour l'ensemble des volontaires analysé est de 20,8. Après 60 jours d'utilisation, le score moyen MASI est de 13,3
Analyse statistique MASI : un test d'égalité de Wilcoxon sur échantillons appariés a été effectué pour déterminer si les moyennes observées entre chaque temps d'étude étaient statistiquement différentes.
On observe donc une différence statistiquement significative (p=0,0006), avec diminution du score clinique MASI de 36,06 % au $60^{ème}$ jour d'utilisation.
La réduction globale de l'hyperpigmentation a été évaluée, au bout de 60 jours, en fonction des différents critères cliniques, par le dermatologue.
Au bout de 60 jours le dermatologue juge que l'hyperpigmentation a été réduite de façon "satisfaisante" ou "très satisfaisante" dans 42,1 % des cas.

B) Evolution de l'index mélanique

Pour chaque côté traité l'index mélanique a été mesuré au niveau de zones symétriques ou similaires, présentant une hyperpigmentation, ceci pour étudier l'effet dépigmentante de chaque produit. De plus l'index mélanique a été mesuré sur une zone de peau saine normalement pigmentée afin de disposer d'une référence de base.
Les résultats des mesures effectuées à chaque temps de mesure et sur chaque sujet ont fait l'objet d'une moyenne par zone (zones traitées, zones de peau saine).
A T0 les index mélaniques mesurés sur les zones hyperpigmentées et sur la peau saine sont différents au plan statistique (p<0,0001), et la différence entre les 2 mesures est de 20,29 (unités arbitraires).
Les résultats moyens observés pour la peau saine témoin sont les suivants :

| | |
|---|---|
| à T0 la valeur moyenne de l'index mélanique est de | 500,89 |
| à T1 la valeur moyenne de l'index mélanique est de | 500,08 |

En comparant la valeur de l'index mélanique en peau saine entre T0 et T1 on n'observe pas de différence

significative (p = 0,6149).

Les résultats moyens observés pour les zones hyperpigmentées traitées sont les suivants :

| | |
|---|---|
| à T0 la valeur moyenne de l'index mélanique est de | 521,18 |
| à T1 la valeur moyenne de l'index mélanique est de | 513,92 |

Entre T0 et T1 on observe une différence significative (p=0,0066) entre la valeur de l'index mélanique mesurée sur les zones hyperpigmentées : la différence entre la mesure à T0 et la mesure à T1 est de 7,26 (unités arbitraires) et la valeur de l'index mélanique est diminuée de 1,39 %.

Par contre à T1 il subsiste une différence significative (p=0,0003) entre les index mélaniques mesurés sur les zones hyperpigmentées et sur la peau saine. La différence entre les mesures à T1 est de 13,84 (unités arbitraires), alors qu'elle était de 20,29 à T0.

C) Tolérance

L'étude des signes fonctionnels au niveau de chaque côté du visage, sensations de tiraillements, de picotements, de brûlures, ne montre aucune variation significative.

D) Auto-estimation des volontaires :

Entre T0 et T1 (60 jours), il existe une différence statistiquement significative pour les signes auto-estimés suivants (test de Wilcoxon) :

| | |
|---|---|
| L'intensité de la couleur des tâches brunes, qui diminue de | 28,1% |
| La peau est plus claire, avec une augmentation de | 63,1% |
| Le teint a plus d'éclat, avec une augmentation de | 90,7% |
| Le teint est plus uniforme, avec une augmentation de | 73,9% |
| Le teint est plus transparent, avec une augmentation de | 83, 4% |

En conséquence, une composition cosmétique dépigmentante selon l'invention, comprenant OX100 en tant que principe actif dépigmentant, est efficace et bien acceptée dermatologiquement sur les lésions hyperpigmentées de type mélasma.

**Exemple 8 :** Effets de l'OX100 sur la pigmentation cutanée

L'effet de l'OX100 a été étudié sur la production de mélanine par des mélanocytes humains normaux en co-culture (NHEM-NHEK ; M/K) irradiés ou non.

Principe et realisation des essais :

Principe:

Des co-cultures de mélanocytes/kératinocytes (NHEM-NHEK ; M/K) sont incubées pendant 240 heures, en absence ou en présence de l'OX100. La quantité de mélanine présente dans les cellules à la fin de l'incubation est mesurée par spectrophotométrie.

Protocole :

1) Les co-cultures M/K sont ensemencées en milieu sans PMA (phorbol myristate acétate) et amenées à 60-80% de confluence pour l'essai.
2) Trois séries de culture identiques sont préparées afin de mesurer :

    La cytotoxicité, par un test au MTT (sel de tétrazolium) et une observation morphologique des cellules.
    La quantité de mélanine.

La quantité de protéines totales (afin de rapporter la quantité de mélanine à la quantité de protéines totales).

3) les conditions de traitement sont les suivantes :

- OX100 à trois concentrations :$2.10^{-6}$, $10^{-5}$,$2.10^{-5}$ M.
- Acide Kojique (molécule de référence dépigmentante).
- IBMX (3-isobutyl-1-méthylxanthine, molécule de référence pro-pigmentante).

Chaque condition de traitement est réalisée en triplicat. Les co-cultures sont incubées pendant 240 heures en présence ou en absence des composés ci-dessus.

Une irradiation par les ultraviolets (25 mJ/$cm^2$ d'UVB + 300 mJ/$cm^2$ d'UVA) est effectuée quatre fois par jour pendant quatre jours consécutifs.

A la fin du traitement, les tapis cellulaires sont lavés. Après lyse des cellules, les cristaux de mélanine sont extraits et solubilisés. La mélanine est quantifiée par mesure de la DO (densité optique) à 450 nm et par comparaison à une gamme étalon de mélanine.

Résultats :

Etude de la viabilité:

L'OX100 ne présente aucune toxicité sur les co-cultures M/K aux trois concentrations testées ( même remarque pour les molécules de références), les résultats sont donnés dans le tableau 8 suivant :

Tableau 8 : pourcentage de viabilité (test au MTT)

| Traitement | Concentration | Sans UV | Avec UV |
|---|---|---|---|
| **Témoin** | - | 100 | 100 |
| **Acide kojique** | 0,0156 mg/ml | 102 | 99 |
| **IBMX** | 200 μM | 118 | 111 |
| **OX100** | $2.10^{-5}$M | 115 | 105 |
| | $10^{-5}$M | 108 | 99 |
| | $2.10^{-6}$M | 102 | 97 |

Etude d'efficacité :

L'effet du produit sur la production de la mélanine a été évalué dans les co-cultures M/K, dans les conditions irradiées ou non (tableau 9).

Tableau 9 : pourcentage de la variante de la quantité de mélanine par rapport au temoin

| Traitement | Concentration | Sans UV | Avec UV |
|---|---|---|---|
| **Contrôle** | - | 100 | 100 |
| **IBMX** | 200μM | 118* (+15%) | 114* (+14%) |
| **Acide kojique** | 15,6 μg/ml | 74* (-35%) | 83* (-20%) |
| **OX100** | $2.10^{-5}$M | 89* (-12%) | 104 (+4%) |
| | $10^{-5}$M | 104 (+4%) | 99 (-1%) |
| | $2.10^{-6}$M | 102 (-1%) | 100 (0%) |

* résultats statistiquement différents du contrôle (p<0,01, test de Dunnett)

l'OX100 diminue significativement (P<0,01) la synthèse de mélanine dans les co-cultures non irradiées, environ 12% d'inhibition à $2.10^{-5}$ M.

L'OX100 peut donc avoir un effet éclaircissant sur la peau normale.
Sur le modèle d'étude irradié, le produit OX100 neutralise l'effet des ultraviolets. En effet, aucune différence significative n'est observée entre le témoin et les traitements par le produit à l'essai. Il n'y a pas d'augmentation de la mélanine sous UV, la peau à la même couleur que le témoin non irradié.

Conclusion :

L'OX100 peut avoir un intérêt dermo-cosmétique en tant que produit dépigmentant, pour le traitement de l'hyperpigmentation cutanée, par sa capacité à neutraliser la surproduction de mélanine induite par les ultraviolets mais également en inhibant sa synthèse dans les conditions non irradiées.

## Revendications

1. Utilisation cosmétique d'au moins une oxazoline, à titre de principe actif dépigmentant, dans une composition dépigmentante.

2. Utilisation cosmétique selon la revendication 1, **caractérisée en ce que** la composition est destinée à réduire et/ou supprimer et/ou prévenir les taches de pigmentation.

3. Utilisation cosmétique selon la revendication 1 ou 2, **caractérisée en ce que** la composition est destinée à améliorer l'uniformité et la clarté du teint.

4. Utilisation cosmétique selon la revendication 1 ou 2 **caractérisée en ce que** ladite oxazoline répond aux formules générales suivantes:

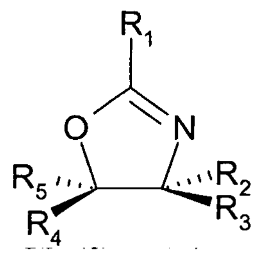

Type 1

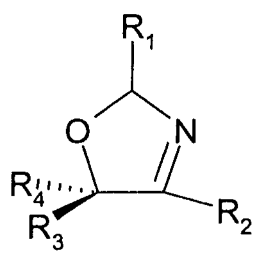

Type 2

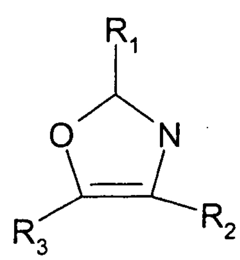

Type 3

dans laquelle $R_1$ représente un groupe alkyle en $C_1$-$C_{40}$, linéaire ou ramifié, saturé ou insaturé, comprenant éventuellement une ou plusieurs insaturation(s) éthylénique(s) ainsi que un ou plusieurs substituant(s) choisi(s) dans le groupe formé par les radicaux hydroxy (OH) et alcoxy en $C_1$-$C_6$ ($OC_1$-$C_6$) ;
$R_2$, $R_3$, $R_4$ et $R_5$ représentent, de manière indépendante, un atome d'hydrogène, un radical hydroxy, ou un groupe alkyle en $C_1$-$C_{30}$, linéaire ou ramifié, saturé ou insaturé, comprenant éventuellement une ou plusieurs insaturations éthyléniques ainsi que un ou plusieurs substituant(s) choisi(s) dans le groupe formé par les radicaux hydroxy (OH), alcoxy en $C_1$-$C_6$ ($OC_1$-$C_6$) et alcoxy en $C_1$-$C_6$ carbonyles ($COOC_1$-$C_6$).

5. Utilisation cosmétique selon l'une quelconque des revendications précédentes, **caractérisée en ce que** ladite oxazoline est une oxazoline de type 1 sélectionnée dans le groupe composé de la 2-undécyl-4-hydroxyméthyl-4-méthyl-1,3-oxazoline, de la 2-undécyl-4,4-diméthyl-1,3-oxazoline, de la (E)-4,4-diméthyl-2-heptadéc-8-ényl-1,3-oxazoline, de la 4-hydroxyméthyl-4-méthyl-2-heptadécyl-1,3-oxazoline, la (E)-4-hydroxyméthyl-4-méthyl-2-heptadéc-8-ényl-1,3-oxazoline, la 2-undécyl-4-éthyl-4-hydroxyméthyl-1,3-oxazoline.

6. Utilisation cosmétique selon l'une quelconque des revendications précédentes, **caractérisée en ce que** ladite oxazoline est la 2-undécyl-4,4-diméthyl-1,3-oxazoline de formule:

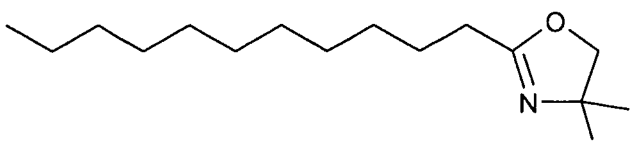

7. Utilisation selon l'une quelconque des revendications précédentes, **caractérisée en ce que** la composition comprend entre 0,01 et 10% en poids d'oxazoline, par rapport au poids total de la composition et un milieu cosmétiquement acceptable.

8. Utilisation selon l'une quelconque des revendications précédentes, **caractérisée en ce que** la composition comprend également un autre principe actif dépigmentant.

9. Utilisation selon l'une quelconque des revendications précédentes, **caractérisée en ce que** la composition comprend en outre au moins un filtre solaire organique ou minéral.

10. Méthode de traitement cosmétique pour réduire et/ou supprimer les taches de pigmentation, **caractérisée en ce que** l'on applique par voie topique une composition comprenant au moins une oxazoline.

11. Méthode selon la revendication 10, **caractérisée en ce que** les taches de pigmentation sont des tâches de vieillesse, ou des tâches solaire, ou des tâches liées à une phototoxicité ou des chloasmas.

12. Méthode de traitement cosmétique pour éclaircir la peau, **caractérisée en ce que** l'on applique par voie topique une composition comprenant au moins une oxazoline.

13. Utilisation d'au moins une oxazoline pour la préparation d'un médicament actif comme dépigmentant.

## Claims

1. The cosmetic use of at least one oxazoline as a depigmenting active ingredient, in a depigmenting composition.

2. The cosmetic use according to claim 1, **characterized** such that the composition is for reducing and / or eliminating and / or preventing pigmentation spots.

3. The cosmetic use according to claim 1 or 2, **characterized** such that the composition is for improving skin color uniformity and lightness.

4. The cosmetic use according to claim 1 or 2, **characterized** such that said oxazoline is of the following general formulas:

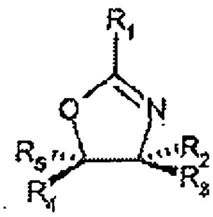

Type 1

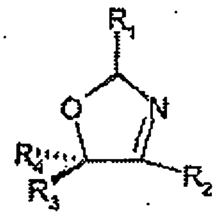

Type 2

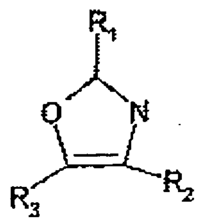

Type 3

in which $R_1$ represents a linear or branched, saturated or unsaturated $C_1$-$C_{40}$ alkyl group optionally comprising one or more ethylene unsaturations as well as one or more substituents chosen from the group formed by hydroxyl (OH) and $C_1$-$C_6$ ($OC_1$-$C_6$) alkoxy radicals; $R_2$, $R_3$, $R_4$ and $R_5$ represent, independent of each other, a hydrogen atom, a hydroxyl radical, or a linear or branched, saturated or unsaturated $C_1$-$C_{30}$ alkyl group optionally comprising one or more ethylene unsaturations as well as one or more substituents chosen from the group formed by hydroxyl (OH), $C_1$-$C_6$ ($OC_1$-$C_6$) alkoxy radicals and C1-C6 alkoxy carbonyl ($COOC_1$-$C_6$) radicals.

5. The cosmetic use according to anyone of the preceding claims, **characterized** such that said oxazoline is an oxazoline of type I chosen from the group comprised of 2-undecyl-4-hydroxymethyl-4-methyl-1,3-oxazoline; 2-undecyl-4,4-dimethyl-1,3-oxazoline; (E)-4,4-dimethyl-2-heptadec-8-enyl-1,3-oxazoline; 4-hydroxymethyl-4-methyl-2-heptadecyl-1,3-oxazoline; (E)-4-hydroxymethyl-4-methyl-2-heptadec-8-enyl-1,3-oxazoline; 2-undecyl-4-ethyl-4-hydroxymethyl-1,3-oxazoline.

6. The cosmetic use according to anyone of the preceding claims, **characterized** such that said oxazoline is 2-undecyl-4,4-dimethyl-1,3-oxazoline of formula:

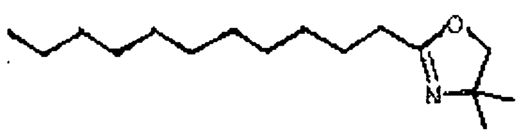

7. The cosmetic use according to anyone of the preceding claims, **characterized** such that the composition comprises between 0.01% and 10% by weight of oxazoline compared to the total weight of the composition and a cosmetically acceptable medium.

8. The cosmetic use according to anyone of the preceding claims, **characterized** such that the composition comprises another depigmenting active ingredient.

9. The cosmetic use according to anyone of the preceding claims, **characterized** such that the composition comprises in addition at least one organic or mineral sun filter.

10. A method of cosmetic treatment for reducing and / or eliminating pigmentation spots, **characterized** such that a composition comprising at least one oxazoline is applied by topical route.

11. A method according to claim 10, **characterized** such that the pigmentation spots are age spots, solar spots or spots related to phototoxicity or chloasma.

12. A method of cosmetic treatment for lightening the skin, **characterized** such that a composition comprising at least one oxazoline is applied by topical route.

13. The use of at least one oxazoline for the preparation of an active medicament as a depigmenting agent.

### Patentansprüche

1. Kosmetische Verwendung mindestens eines Oxazolins als depigmentierender Wirkstoff in einer Depigmentierungszusammensetzung.

2. Kosmetische Verwendung nach Anspruch 1, **dadurch gekennzeichnet, dass** die Zusammensetzung zur Verringerung und/oder Unterdrückung und/oder Verhinderung von Pigmentflecken bestimmt ist.

3. Kosmetische Verwendung nach Anspruch 1 oder 2, **dadurch gekennzeichnet, dass** die Zusammensetzung zur Verbesserung der Ebenmäßigkeit und Klarheit des Teints bestimmt ist.

4. Kosmetische Verwendung nach Anspruch 1 oder 2, **dadurch gekennzeichnet, dass** das Oxazolin die folgenden allgemeinen Formeln aufweist:

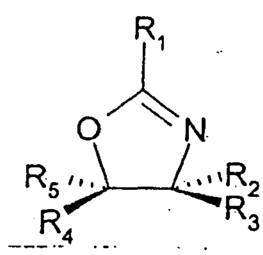

Typ 1

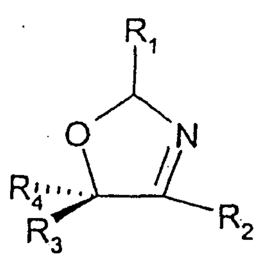

Typ 2

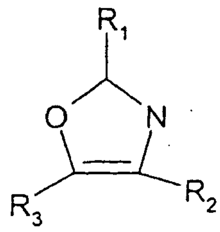

Typ 3

wobei $R_1$ einen geradkettigen oder verzweigten, gesättigten oder ungesättigten $C_1$-$C_{40}$-Alkylrest bedeutet, welcher gegebenenfalls einfach oder mehrfach ethylenisch ungesättigt ist sowie einen oder mehrere Substituenten, ausgewählt aus Hydroxygruppen (OH) und $C_1$-$C_6$-Alkoxyresten ($OC_1$-$C_6$), umfasst,
$R_2$, $R_3$, $R_4$ und $R_5$ unabhängig voneinander ein Wasserstoffatom, eine Hydroxygruppe oder einen linearen oder verzweigten, gesättigten oder ungesättigten $C_1$-$C_{30}$-Alkylrest, welcher gegebenenfalls einfach oder mehrfach ethylenisch ungesättigt ist sowie einen oder mehrere Substituenten, ausgewählt aus Hydroxygruppen (OH), $C_1$-$C_6$-Alkoxyresten ($OC_1$-$C_6$) und $C_1$-$C_6$-Carbonylalkoxyresten ($COOC_1$-$C_6$), umfasst, bedeuten.

**5.** Kosmetische Verwendung nach einem der vorhergehenden Ansprüche, **dadurch gekennzeichnet, dass** das Oxazolin ein Oxazolin vom Typ 1 ist, das aus 2-Undecyl-4-hydroxymethyl-4-methyl-1,3-oxazolin, 2-Undecyl-4,4-dimethyl-1,3-oxazolin, (E)-4,4-Dimethyl-2-heptadec-8-enyl-1,3-oxazolin, 4-Hydroxymethyl-4-methyl-2-heptadecyl-1,3-oxazolin, (E)-4-Hydroxymethyl-4-methyl-2-heptadec-8-enyl-1,3-oxazolin, 2-Undecyl-4-ethyl-4-hydroxymethyl-1,3-oxazolin ausgewählt ist.

**6.** Kosmetische Verwendung nach einem der vorhergehenden Ansprüche, **dadurch gekennzeichnet, dass** das Oxazolin 2-Undecyl-4,4-dimethyl-1,3-oxazolin der Formel:

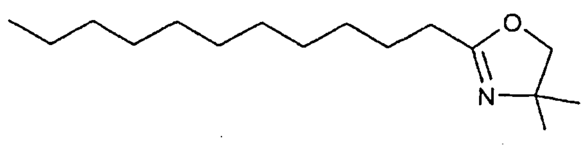

ist.

**7.** Verwendung nach einem der vorhergehenden Ansprüche, **dadurch gekennzeichnet, dass** die Zusammensetzung zwischen 0,01 und 10 Gew.-% Oxazolin, bezogen auf das Gesamtgewicht der Zusammensetzung, und ein kosmetisch verträgliches Medium umfasst.

**8.** Verwendung nach einem der vorhergehenden Ansprüche, **dadurch gekennzeichnet, dass** die Zusammensetzung weiter einen anderen Depigmentierungs-Wirkstoff umfasst.

**9.** Verwendung nach einem der vorhergehenden Ansprüche, **dadurch gekennzeichnet, dass** die Zusammensetzung ferner mindestens einen organischen oder mineralischen Sonnenfilter umfasst.

**10.** Kosmetisches Behandlungsverfahren zur Verringerung und/oder Unterdrückung von Pigmentflecken, **dadurch gekennzeichnet, dass** eine mindestens ein Oxazolin umfassende Zusammensetzung topisch aufgebracht wird.

**11.** Verfahren nach Anspruch 10, **dadurch gekennzeichnet, dass** die Pigmentflecken Altersflecken oder Sonnenflecken oder mit einer Phototoxizität oder Chloasmen in Verbindung stehende Flecken sind.

**12.** Kosmetisches Behandlungsverfahren zur Aufhellung der Haut, **dadurch gekennzeichnet, dass** eine mindestens ein Oxazolin umfassende Zusammensetzung topisch aufgebracht wird.

**13.** Verwendung mindestens eines Oxazolins zur Herstellung eines Medikaments, das als Depigmentierungsmittel wirksam ist.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- FR 2834216
- FR 2856294
- US 4876249 A

### Littérature non-brevet citée dans la description

- **KIMBROUGH-GREEN et al.** *Arch Dermatol,* 1994, vol. 130, 727-733